# EUROPÄISCHE PATENTSCHRIFT

(11) **EP 1 627 609 B1**
(45) Veröffentlichungstag und Bekanntmachung des Hinweises auf die Patenterteilung: **17.10.2007**
(21) Anmeldenummer: 04405522.6
(22) Anmeldetag: 20.08.2004
(51) Int. Cl.: A61B 17/64, F16B 7/04

(54) **Klemmelement zum Klemmen von mehreren stabförmigen Elementen**
Clamping element for clamping of several rod-like elements
Elément de serrage pour accrocher ensemble plusieurs tiges

(43) Veröffentlichungstag der Anmeldung: 22.02.2006
(73) Patentinhaber: Stryker Trauma SA, 2545 Selzach (CH)
(72) Erfinder: Thomke, Roland, 4512 Bellach (CH); Burgherr, Vinzenz, 3005 Bern (CH); Lutz, Christian, 4500 Solothurn (CH); Fankhauser, Damian, 3007 Bern (CH); Dransfeld, Clemens, 5702 Niederlenz (CH); Fischer, René, 8057 Zürich (CH)
(74) Vertreter: Liebetanz, Michael

(56) Entgegenhaltungen:
- EP-A- 0 700 664
- WO-A-97/35527
- WO-A-20/05085658
- DE-A- 19 753 010
- SU-A- 1 627 161
- US-A- 4 293 176
- US-A- 5 728 096
- US-A- 5 891 144

## Beschreibung

### Technisches Gebiet der Erfindung

Die Erfindung betrifft ein Klemmelement zum Klemmen von mehreren stabförmigen Elementen, beispielsweise Stiften, und insbesondere ein Klemmelement zur Stabilisierung von Knochenbrüchen, gemäss dem Oberbegriff des Anspruchs 1.

### Stand der Technik

Die US 6,565,564 beschreibt ein solches Klemmelement zum Klemmen von mehreren Stiften, das über zwei gegenüberliegende Klemmbacken mit einer Mehrzahl von parallelen Rillen zur Aufnahme einer entsprechenden Anzahl von stabförmigen Elementen verfügt. Es sind zwei quer zu den Rillen ausgerichtete Bohrungen in den Klemmbacken vorgesehen, durch die jeweils eine Verbindungsschraube einsetzbar ist.

Ein solches Klemmelement hat den Vorteil, dass mehrere nebeneinander angeordnete Stifte einen Bruch über der Bruchstelle definiert richten können, wobei dabei die Verbindungsschraube von einer Klemmbacke ausgehend in eine in der anderen Klemmbacke eingesetzten innengewindete Mutter eingeschraubt wird, um die Klemmbacken zu schliessen.

Es ist ein Nachteil der bekannten Vorrichtung, dass das Klemmelement durch Anziehen von verschiedenen Schrauben zeitlich nur langsam geschlossen werden kann.

Es sind zwischen den Klemmbacken und um die beiden Verbindungsschrauben Spiralfedern eingesetzt, um die Klemmbacken im Ruhezustand auseinander zu drücken und das Klemmelement leichter auf die Stifte einsetzen zu können.

Aus der EP 0 700 664 A1 ist ein Klemmelement für zwei Stäbe bekannt, die jeweils zwischen zwei unterschiedlichen Klemmbackenpaaren klemmbar sind. Dabei ist eine zentrale Feder zwischen den beiden Klemmbackenpaaren vorgesehen, so dass die Stäbe seitlich gegen die Wirkung der zentralen Feder einklipsbar sind. Die EP 0 700 664 A1 zeigt zudem ein Klemmelement zum Klemmen von mehreren Stiften zwischen einem gemeinsamen Klemmbackenpaar.

Die US 4,293,176 zeigt einen Verbinder für zwei einzulegende elektrische Kabel, bei dem die Klemmbacken durch eine einzige Schraube auf den Kabeln verriegelbar sind. Beim Anziehen werden um die Schraube herum liegende Federelemente aufeinanderzu bewegt, zusammengedrückt und übernehmen die Haltefunktion anstelle der nur aus dünnen Platten bestehenden Klemmbacken.

Ausgehend von diesem Stand der Technik ist es eine Aufgabe der vorliegenden Erfindung ein einfaches Klemmelement anzugeben, welches eine schnellere Fixierung von eingesetzten Stiften ermöglicht.

Ein weiteres Ziel der Erfindung ist es, ein kostengünstiges leichtes Einwegklemmelement zu schaffen, insbesondere aus Kunststoff im Spritzguss.

### Zusammenfassung der Erfindung

Diese Aufgabe wird erfindungsgemäss für ein Klemmelement der eingangs genannten Art mit den kennzeichnenden Merkmalen des Anspruchs 1 gelöst.

Dadurch, dass zwei Klemmbacken über zwei Federelemente zu einem vorteilhafterweise einstückigen Klemmelement verbunden werden, wobei durch einfaches Betätigen der Federelemente die Klemmbacken räumlich etwas voneinander entfernt, die Stifte positioniert und schliesslich blockiert werden können, wird ein solches Klemmelement plötzlich in einfacher Weise betätigbar.

Weitere vorteilhafte Ausführungsbeispiele sind in den Unteransprüchen niedergelegt.

### Kurze Figurenbeschreibung

Die Erfindung wird nun unter Bezugnahme auf die Zeichnungen an Hand von Ausführungsbeispielen beispielhaft näher beschrieben. Es zeigen:
- Fig. 1: eine perspektivische Explosionsansicht eines ersten Ausführungsbeispiels eines Klemmelementes gemäss der Erfindung,
- Fig. 2: eine Draufsicht auf ein zweites Ausführungsbeispiel eines Klemmelementes ähnlich wie Fig. 1,
- Fig. 3: eine perspektivische Ansicht eines Federelementes des Klemmelementes nach Fig. 1 oder 2,
- Fig. 4: eine perspektivische Ansicht einer Klemmbacke des Klemmelementes nach Fig. 1 oder Fig. 2,
- Fig. 5: eine perspektivische Ansicht eines dritten Ausführungsbeispiels eines Klemmelementes gemäss der Erfindung,
- Fig. 6: eine perspektivische Ansicht einer Klemmbacke des Klemmelementes nach Fig. 5,
- Fig. 7: eine Draufsicht auf das Klemmelement nach Fig. 5,
- Fig. 8: eine perspektivische Ansicht eines Federelementes des Klemmelementes nach Fig. 5,
- Fig. 9: eine Draufsicht auf ein viertes Ausführungsbeispiel eines Klemmelementes gemäss der Erfindung ohne Stifte,
- Fig. 10: die Draufsicht nach Fig. 9 mit eingesetzten Stiften,
- Fig. 11: ein Querschnitt durch eine zu Fig. 4 ähnliche Klemmbacke,
- Fig. 12: eine Draufsicht auf die Klemmbacke nach Fig. 11,
- Fig. 13: eine vergrösserte Seitenansicht der Klemmbacke nach Fig. 11,
- Fig. 14: eine vergrösserte Draufsicht auf die Klemmbacke nach Fig. 11,
- Fig. 15: ein Querschnitt durch eine Klemmbacke mit ansetzbarem Stab für ein Federelement nach Fig. 8,
- Fig. 16: eine Draufsicht auf die Klemmbacke nach Fig. 15,
- Fig. 17: eine Seitenansicht des Klemmelementes nach Fig. 1,
- Fig. 18: eine Draufsicht auf das Klemmelement nach Fig. 1,
- Fig. 19: eine perspektivische Ansicht eines Federelementes nach einem weiteren Ausführungsbeispiel für eine Klemmbacke nach Fig. 4,
- Fig. 20: eine Seitenansicht des Federelementes nach Fig. 19,
- Fig. 21: eine Seitenansicht des Federelementes nach Fig. 19,
- Fig. 22: eine teilweise geschnittene Seitenansicht einer Verriegelungsschraube, einer Mutter und einer Sicherungsschraube für eine Klemmbacke,
- Fig. 23: eine Seitenansicht auf ein fünftes Ausführungsbeispiel eines Klemmelementes gemäss der Erfindung ohne Stifte,
- Fig. 24: eine Draufsicht auf das Klemmelement nach Fig. 23,
- Fig. 25: eine schematische Seitenansicht auf ein sechstes Ausführungsbeispiel eines Klemmelementes gemäss der Erfindung ohne Stifte, und
- Fig. 26: eine schematische Seitenansicht auf ein siebtes Ausführungsbeispiel eines Klemmelementes gemäss der Erfindung ohne Stifte.

### Ausführliche Beschreibung der bevorzugten Ausführungsbeispiele

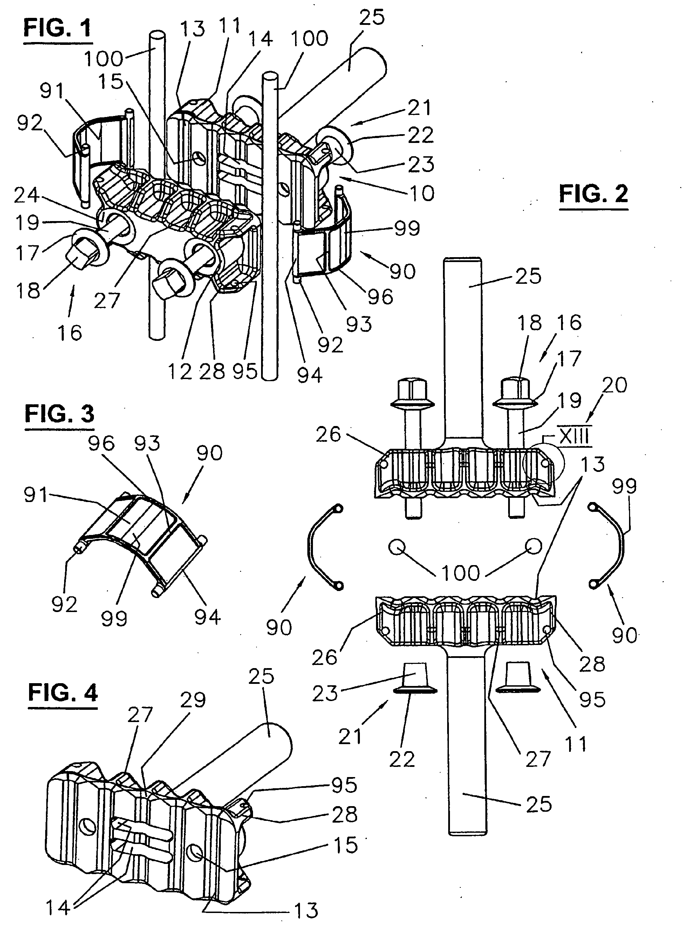

Die Fig. 1 zeigt eine perspektivische Explosionsansicht eines ersten Ausführungsbeispiels eines Klemmelementes 10 gemäss der Erfindung. Das Klemmelement 10 besteht aus zwei Klemmbacken 11 und 12. Diese Klemmbacken 11 und 12 sind auf der einander zugewandten Seite im wesentlichen gleich ausgestaltet. Nur auf der abgewandten Seite verfügt die Klemmbacke 11 über einen angeformten Stab 25, während die Klemmbacke 12 keine nach aussen führende Verbindung aufweist.

Die Klemmbacken 11 und 12 verfügen über hier fünf parallele Rillen 13. Es können auch mehr oder weniger Rillen 13 vorgesehen sein. Verschiedene Rillen 13 können auch in einem Winkel zueinander verlaufen, beispielsweise in einem Winkel von 30 Grad. Die Rillen 13 können auch teilweise parallel zueinander und teilweise in verschiedenen Winkeln zueinander ausgerichtet sein.

Die Klemmbacke 11 verfügt auf der Innenseite gegenüber dem Stab 25 über zwei quer zu den Rillen 13 verlaufende Schlitze 14 mit fast parallelen Seitenwänden, die in einer Klemmbacke selber noch enden. In den Klemmbacken 11 und 12 sind seitlich jeweils zwei Bohrungen 15 vorgesehen, die an der Aussenseite über eine konische Aufnahme 24 verfügen. In die Bohrung 15 der einen Klemmbacke, hier 12, wird von einer Seite eine Schraube 16 mit konisch komplementären Flansch 17 und einem Vierkant-Kopf 18 eingesetzt und von der Bohrung 15 der anderen Klemmbacke, hier 11, wird eine Mutter 21 mit konischem Flansch 22 und Hülse 23 eingesetzt. Der gewindete Schraubenschaft 19 greift durch die Bohrungen 15 in die Hülse 23 der Mutter 21 und gestattet ein Aufeinander zu bewegen der Klemmbacken 11 und 12. Die Bohrung 15 und die komplementäre Hülse 23 können beispielsweise auch zylindrisch ausgestaltet sein.

Die Kombination Schraube 16 und Mutter 21 kann auch nur durch eine einzige, in der anderen Klemmbacke einzuschraubenden Schraube ersetzt werden. Es kann ein Gewinde in der Bohrung 15 vorgesehen sein oder die Schraube weist ein selbstschneidendes Gewinde auf. Es kann ganz allgemein ein Verriegelungselement vorgesehen sein, bei dem es sich auch um eine Hebelverriegelung oder einen Bajonettverschluss handeln kann. Zu diesen Verriegelungselementen können auch Unterlegscheiben oder Zahnscheiben hinzutreten, die in den Zeichnungen aus vereinfachungsgründen nicht dargestellt sind. Die beiden gegeinander einzusetzenden Klemmbacken (beispielsweise [11 und 11] oder [11 und 12] oder [12 und 12]) können im gleichen Werkzeug hergestellt werden, insbesondere einem Mehrfachwerkzeug mit fast identischen Hohlformen, in dem die Bohrung 15 der einen Spritzgussform für die direkte Aufnahme einer Schraube mit einem geringeren Durchmesser der Bohrung (oder des Gewindes) ausgestaltet ist.

Zwischen den Klemmbacken 11 und 12 werden stabförmige Elemente, beispielsweise Stifte 100, eingesetzt. Hier sind zwei Stifte 100 dargestellt.

Die Klemmbacken 11 und 12 weisen im Bereich der Schraubenaufnahmen 24 Vollmaterial auf. Zu den Rändern hin sind an den Längsseiten nur drei Rippen 27 vorgesehen. An den Ecken jeder Klemmbacke 11 oder 12 sind insgesamt vier Federmontagerippen 28 vorgesehen. Diese Federmontagerippen 28 der Klemmbacken 11 und 12 verfügen jeweils über ein durchgehendes kleines rundes Loch 95 zur Aufnahme eines entsprechendes runden Zapfens 92 eines Federelementes 90. An einer Schmalseite eines Klemmbackens 11 oder 12 sind die beiden Löcher 95 fluchtend angeordnet und liegen parallel zu der Hauptachse der hier vorgesehenen Rillen 13.

Somit ist ein Federelement 90 einsetzbar, dass in der Fig. 3 in seiner vorgespannten Form dargestellt ist, die eine perspektivische Ansicht des Federelementes 90 zeigt, das heisst bei einem Spritzgussteil in seinem entspannten Zustand wären die durch die Zapfen 92 vorgegebenen Achsen parallel näher beieinander. Gleiches gilt für ein Federelement 90 beispielsweise in Gestalt eines gestanzten Metallteils. Es verfügt über jeweils einen Steg 94 an den freien Enden. Der Steg 94 endet selber an beiden Enden in Rundzapfen 92, die in die Löcher 95 eingreifen können. Die beiden Stege 94 sind über zwei flexible seitliche Längsrippen 96 miteinander verbunden. Ferner sind zwei verstärkende Querrippen 93 vorgesehen. Zwischen den genannten Rippen spannen sich Federelementfelder 91 auf. Die Länge und Bogenform des Federelementes 90 ist dergestalt, dass es eingesetzt in die Löcher 95 die beiden Klemmbacken 11 und 12 aufeinander zu bewegt, also schliesst.

Es ist also notwendig, auf die mittleren Flächen als Betätigungsbereiche 99 zu drücken, um die Krümmung des Federelementes 90 auf dem Weg zu den freien Enden zu reduzieren, die beiden Stege 94 weiter voneinander zu entfernen und damit die zugehörigen Klemmbacken 11 und 12 zu öffnen. Beim Loslassen der Federelemente 90 schliesst sich dann das Klemmelement 10 automatisch.

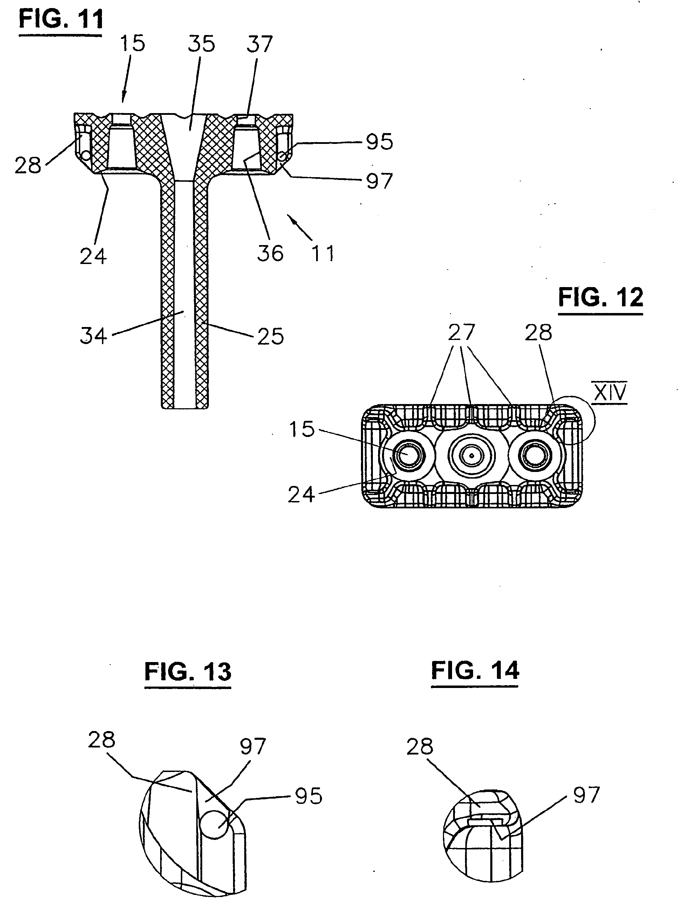

Die Fig. 2 zeigt eine Draufsicht auf ein zweites Ausführungsbeispiel eines Klemmelementes 20 ähnlich wie Fig. 1. Gleiche Merkmale sind jeweils mit gleichen Bezugszeichen versehen. Der Unterschied liegt darin, dass zwei identische Klemmbacken 11 Verwendung gefunden haben. Mit XIII ist ein Ausschnitt bezeichnet, der in der Fig. 13 vergrössert dargestellt wird.

Die Fig. 4 zeigt eine perspektivische Ansicht einer Klemmbacke des Klemmelementes nach Fig. 1 oder Fig. 2. Es ist zu erkennen, dass gegenüber jeder Rille 13 eine Verstärkungsrippe 27 vorgesehen ist. Ferner ist eine Federmontagerippe 28 für die seitlichen Rillen 13 und die Montage der Federelemente 90 vorgesehen. Jede Rille ist zum leichteren Einschieben der Stifte 100 mit einer Anfräsung 29 versehen.

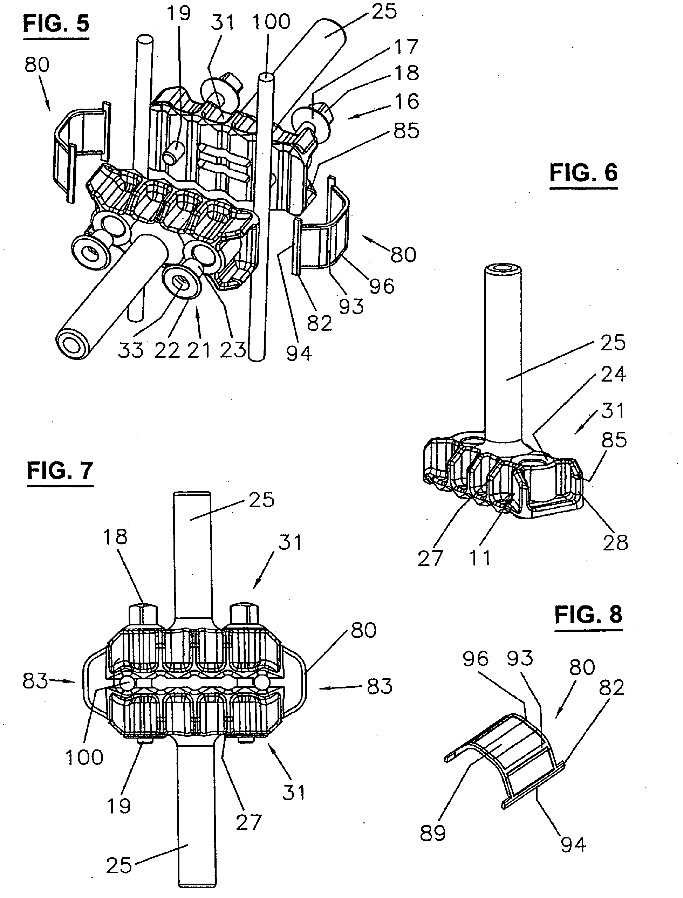

Die Fig. 5 zeigt eine perspektivische Ansicht eines dritten Ausführungsbeispiels eines Klemmelementes 30 gemäss der Erfindung. Die Fig. 6 zeigt eine perspektivische Ansicht einer Klemmbacke 31 des Klemmelementes 30 nach Fig. 5, die Fig. 7 eine Draufsicht auf das Klemmelement nach Fig. 5, und die Fig. 8 eine perspektivische Ansicht eines Federelementes 80 des Klemmelementes 30. Die Klemmbacken 31 sind im wesentlichen gleich zu den Klemmbacken 11 ausgestaltet. Der einzige Unterschied betrifft die Federmontagerippe 28. Diese verfügt über innen angeordnete Schlitze 85. Diese Schlitze 85 sind rechtwinklige geformte Ausnehmungen, die natürlich auch im Spritzguss herstellbar sind.

Das Federelement 80 verfügt über ein ähnliches Federblatt 91, welches von Rippen 93 und 96 umgeben ist. Die seitlichen Stege 94 sind hier rechtwinklig und enden in ebensolchen Rechteckzapfen 82. Durch ein Drücken auf den mittigen Betätigungsbereich 89 des Federblattes 91 in Pfeilrichtung 83 nach Fig. 7 gehen die beiden Klemmbacken 31 auseinander und geben damit die Stifte 100 frei. Diese Stifte 100 könnten durch weitere ergänzt oder in ihrer Lage verändert werden, und so weiter. Vorteilhafterweise helfen die nach aussen erhöhten Rippen 93 und 96, den Betätigungsbereich 89 beispielsweise für die Kuppe eines Daumens und eines Zeigefingers des Benutzers zu begrenzen, so dass er nicht abrutschen kann.

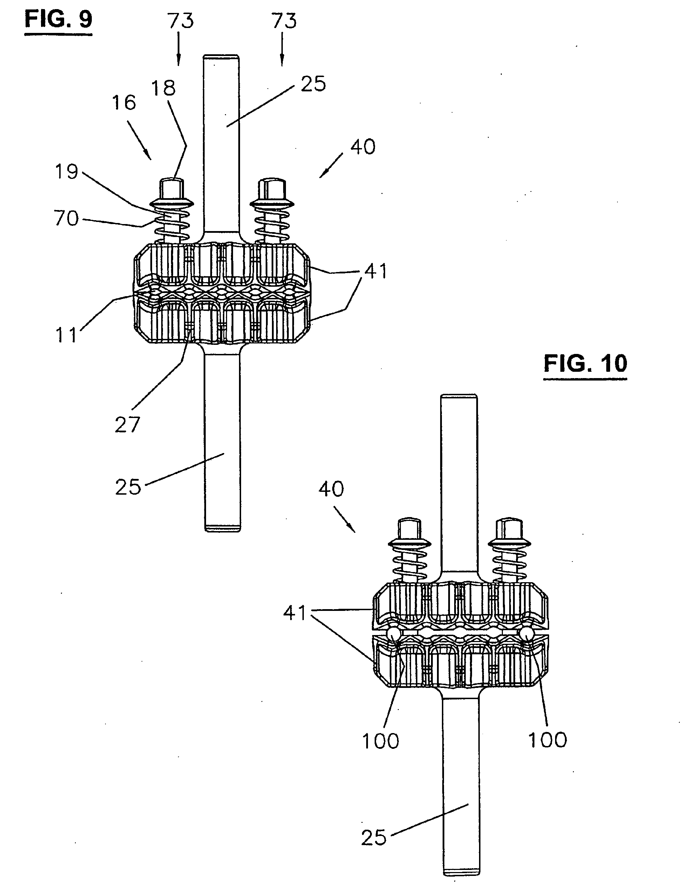

Die Fig. 9 zeigt eine Draufsicht auf ein viertes Ausführungsbeispiel eines Klemmelementes 40 gemäss der Erfindung ohne Stifte und die Fig. 10 zeigt die Draufsicht nach Fig. 9 mit eingesetzten Stiften 100. Bei dem Ausführungsbeispiel nach Fig. 9 sind Federelemente 70, hier Spiralfedern, um die beiden Schrauben 16 angeordnet und stützen sich einerseits auf der Schraubenaufnahme 24 und andererseits auf der Unterseite des Flansches 17 des Schraubenkopfes 18 ab. Somit drücken die Federn 70 die Klemmbacken 41 zusammen. Wenn der in der Zeichnung oberer Stab 25 bereits fixiert ist, kann durch Drücken im Pfeilrichtung 73, beispielsweise auf beide Schraubenköpfe 18, die zeichnerisch untere Klemmbacke 41 von der oberen abgehoben werden, um einen grösseren Zwischenraum zwischen den Rillen 14 zu schaffen und die beiden Stifte 100 einschieben zu können. Die Spiralfedern 70 sind zumeist aus Metall, können jedoch auch aus Kunststoff sein. Es können auch gelochte Federtellerpakete oder andere Federelemente sein.

Die Fig. 11 zeigt einen Querschnitt durch eine zu Fig. 4 ähnliche Klemmbacke 11, wobei zur Übersicht die Fig. 12 eine Draufsicht auf die Klemmbacke 11 nach Fig. 11, Fig. 13 eine vergrösserte Seitenansicht eines Ausschnittes der besagten Klemmbacke 11, und Fig. 14 eine vergrösserte Draufsicht auf denselben Ausschnitt der Klemmbacke 11 nach Fig. 11 zeigt.

Dabei ist zu erkennen, dass die Bohrung 37 im Bereich der Rillen 14 eine zylindrische Bohrung mit einem Durchmesser von beispielsweise 5 Millimeter ist. Sie kann auf der Innenseite über drei, vier oder mehr dünne Rippen von beispielsweise 0,5 Millimeter Höhe aufweisen, um die Schraube 16 zentriert zu führen. Daran schliesst sich ein sich konisch vergrössernder Bohrraum 36 an, beispielsweise bis auf 8 Millimeter ansteigend. Schliesslich mündet die Bohrung 15 in einer Schraubenaufnahme 24 mit einem Durchmesser von beispielsweise 14 Millimetern und am Rand mit einem äusseren Öffnungswinkel von 60 Grad. Der konisch sich vergrössernde Bohrraum 36 kann mit einem konischen Innengewinde ausgestaltet sein, um die Hülse 23 der Mutter 21 sicher aufnehmen zu können. Sie könnte aber auch in einem Presssitz eingedrückt werden.

Die zentrale Bohrung 35 kann die Funktion der Schlitze 14 übernehmen, dass heisst beispielsweise bei der Spritzgusstechnik eine vereinfachte Abformung einsetzen zu können.

Seitlich ist in dem Querschnitt die jeweils hinter der Schnittebene angeordnete Federmontagerippe 28 zu erkennen, wobei der Weg zu dem durchgehenden Loch 95 mit einem sich verjüngenden Absatz 97 ausgestaltet ist, so dass dieser Absatz 97 für den Zapfen 92 eine Führung bietet. Dies ist insbesondere in den Fig. 13 und 14 zu erkennen. Der Absatz 97 ist einen halben Millimeter stark, was zu einer Führungsfunktion ausreichend ist. Bei Einsatz einer geeigneten Bestückungstechnik kann auf dem Absatz 97 auch verzichtet werden.

Die Rillen 14 decken einen Winkelbereich von 120 Grad ab und weisen einen Krümmungsradius von 2 Millimetern auf.

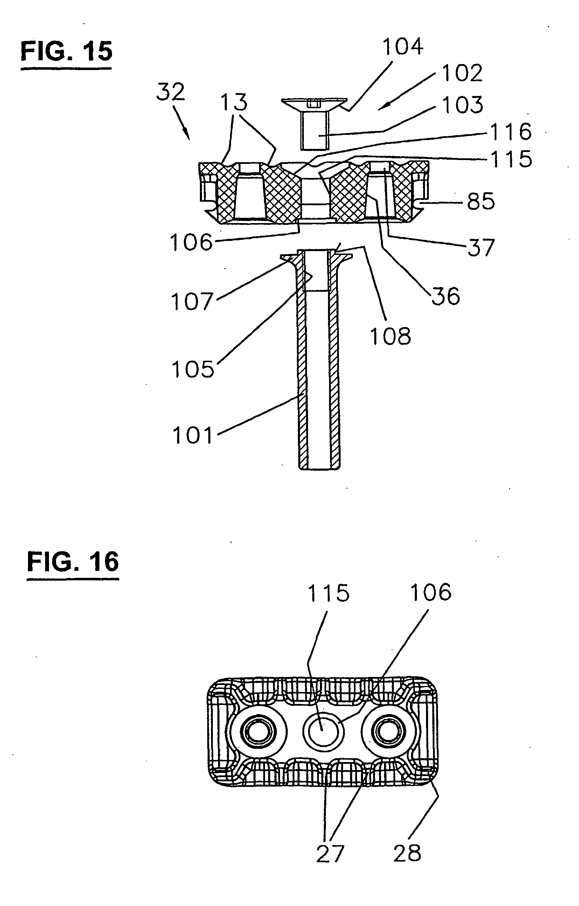

Die Fig. 15 zeigt einen Querschnitt durch eine Klemmbacke 32 ähnlich zu Fig. 6 mit ansetzbarem Stab und für ein Federelement nach Fig. 8 und Fig. 16 zeigt eine Draufsicht auf diese Klemmbacke 32. Die Klemmbacke 32 verfügt über eine Innenbohrung 115 mit einer konischen Mündung 116 auf der den Rillen 13 zugewandten Seite. Auf der gegenüberliegenden Seite verfügt sie über einen einen grösseren Durchmesser aufweisenden Absatz 106.

Dies ermöglicht den versatilen Einsatz der Klemmbacke 32 durch vom Operateur gewünschten Einsatz eines ansetzbaren Stabes 101 in gewünschter Länge. Der ansetzbare Stab 101 ist zumindest im zur Montage vorgesehenen Endbereich hohl und verfügt über ein Innengewinde 105. Er hat hier einen sich verbreiternden Flansch 107 und einen schmaleren Ansatz 108, der in den Absatz 106 einsetzbar ist. Dann kann durch die von der gegenüberliegenden Seite vorzuschiebenden Befestigungsschraube 102 deren Aussengewinde 103 in das Innengewinde 104 eingreifen, wobei sich beim Anziehen der Schraube der konisch verbreiterte Kopf 104 auf der Mündung 116 absetzt. Neben dieser Befestigungsform über eine Schraube 102 können auch andere Verriegelungsformen wie Bajonettverschlüsse oder Hebelverschlüsse vorgesehen sein. Der einsetzbare Stab 101 kann auch selber an seinem einen (oder beiden) Enden anstelle des Flansches 107 über ein Aussengewinde verfügen, welches sich in ein in der Bohrung 115 vorgesehenes Innengewinde oder in eine in die Bohrung 115 von der gegenüberliegenden Seite einsetzbare Hülse einschraubt.

Wesentlich und vorteilhaft für den Operateur ist die Möglichkeit, aus einer Klemmbacke 32 durch Ansetzen eines von ihm gewählten Stabes 101 beliebiger Konfiguration und insbesondere Länge eine für den Einsatz besonders geeignete Klemmbacke herzustellen, die sich von den vorkonfigurierten Klemmbacken 11 oder 41 abhebt.

Die Federmontagerippe 28 verfügt über durchgehende Schlitze 85, in die beispielsweise ein Federelement nach Fig. 8 einsetzbar ist.

Allerdings ist diese Beschreibung so zu verstehen, dass die einzelnen Elemente beliebig miteinander koppelbar sind, also jedes Federelement (Metall, Blattfeder, einzelnes Spritzgussteil, 2K-Spritzgussteil) mit jeder Art von Montage (Schlitz oder loch etc.) und jeder Art von Klemmbacke (Rillenorientierung und/oder Stabmontage) kombinierbar ist.

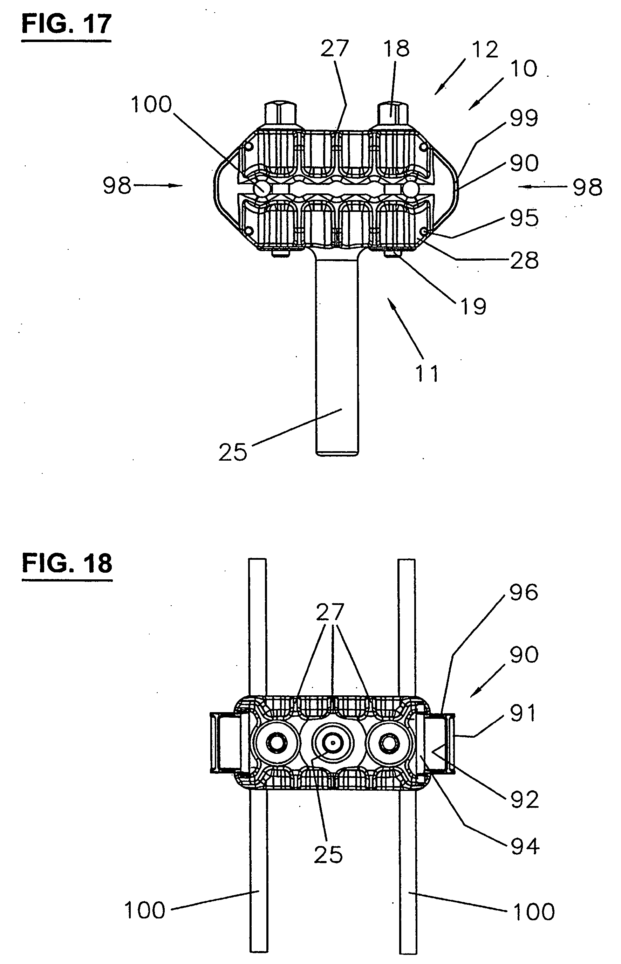

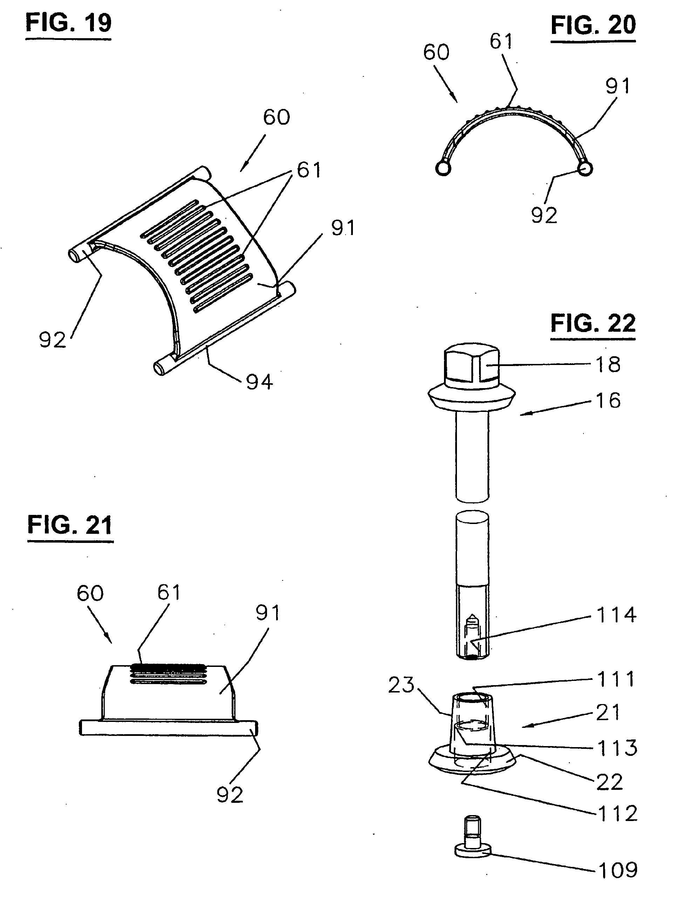

Die Fig. 17 zeigt eine Seitenansicht des Klemmelementes 10 nach Fig. 1 und Fig. 18 eine Draufsicht auf dieses Klemmelement 10. Es ist zu erkennen, dass die Schrauben 16 mit ihrem Schraubenkörper 19 durch die Muttern 21 hindurchtreten. Es wäre auch möglich, dass eine Sicherungsschraube in ein Innengewinde im Schaftende der Schraube 16 eingesetzt wird, wobei dann eine Schulter in der Mutter 21 gewährleistet, dass die Schraube 18 und die Mutter 21 unverlierbar miteinander verbunden sind, wie es in der Fig. 22 dargestellt ist. Bei anderen Ausführungsbeispielen kann die Unverlierbakeit auch durch Taumelnieten der Schraube 16 gewährleistet werden.

In Fig. 17 ist zu erkennen, dass durch beidseitigen Druck auf die Federelemente 90 in Pfeilrichtung 98 die beiden Klemmbacken 11 mehr voneinander gelöst werden und damit die Stifte 100 freigeben.

Die Fig. 19 zeigt eine perspektivische Ansicht eines Federelementes 60 nach einem weiteren Ausführungsbeispiel für eine Klemmbacke 11 nach Fig. 4, Fig. 20 eine Seitenansicht des besagten Federelementes 60 und Fig. 21 eine Seitenansicht dieses Federelementes 60. Hier ist eine im Querschnitt gleichmässig gerundete Federfläche 91 vorgesehen, auf der in der Mitte hier vier Rippen 61 vorgesehen sind, deren Betätigung Durch Druckbeaufschlagung der Betätigung in Pfeilrichtung 73, 83 oder 98 entspricht.

Die Fig. 22 zeigt eine teilweise geschnittene Seitenansicht einer Schraube 16, einer Mutter 21 und einer Sicherungsschraube 109 für ein Klemmelement 10 nach einer der Fig.. Der Konus 23 der Mutter 21 kann glatt sein, er kann aber auch ein Aussengewinde aufweisen. Dann ist vorzugsweise auch die Bohrung 15 mindestens teilweise mit einem Gewinde versehen. Die Hülse 23 der Mutter 21 verfügt über eine durchgehende Bohrung mit einem ersten schmaleren Abschnitt 111, der ein Innengewinde zur Aufnahme des Aussengewindes der Schraube 16 aufweist. Ein daran anschliessender breiter zweiter Abschnitt 112 verfügt über eine Schulter 113, auf der sich der Schraubenkopf der Sicherungsschraube 109 absetzen kann. Die Sicherungsschraube 109 ist dafür vorgesehen, sich in ein Innengewinde 114 in der Spitze der Schraube 16 einzuschrauben. Dabei kann insbesondere vorgesehen sein, beispielsweise durch Klebstoff oder ein anderes Mittel, dass nach einem Vorzusammenbau eines Klemmelementes 10 sich die Schraube 16 nicht mehr aus dem Gewinde 114 lösen lässt, womit die Einheit des Klemmelementes 10 gesichert ist.

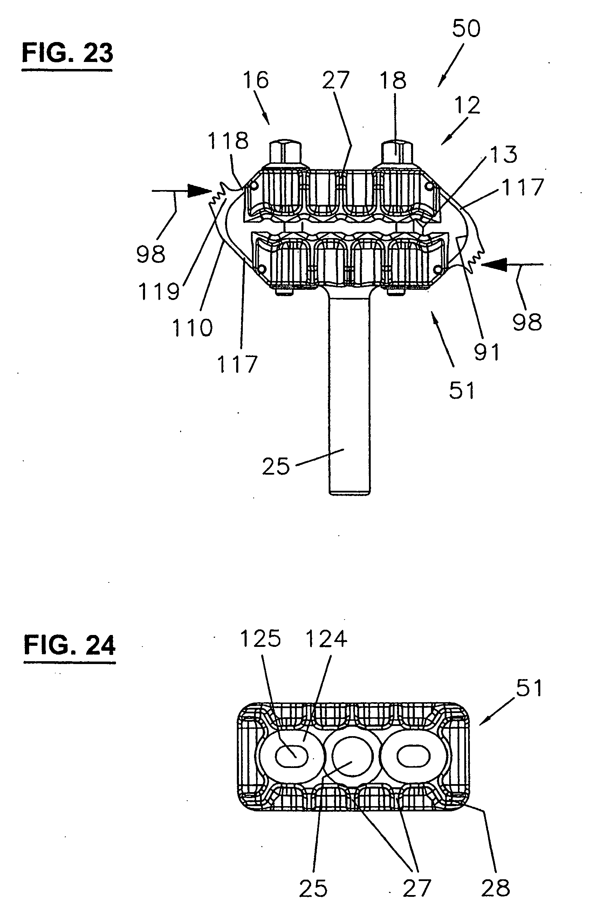

Die Fig. 23 zeigt eine Seitenansicht auf ein fünftes Ausführungsbeispiel eines Klemmelementes 50 gemäss der Erfindung ohne Stifte. Die Fig. 24 zeigt eine Draufsicht auf das Klemmelement 50 nach Fig. 23. Die eine Klemmbacke 12 entspricht beispielsweise der entsprechenden Klemmbacke 12 aus Fig. 1. Ihr gegenüber ist eine andere Klemmbacke 51 angeordnet, an die hier beispielsweise ein Stab 25 angeformt ist. Sie kann aber auch stablos sein oder entsprechend Fig. 15 ausgestaltet sein. Die Klemmbacken 12 und 51 verfügen über die schon beschriebenen durchgehenden kleinen runden Löcher 95 zur Aufnahme eines Federelementes 110. Dieses Federelement 110 ist vorzugsweise asymmetrisch ausgestaltet und verfügt über ein längeres Bein 117 und ein kürzeres Bein 118, die in einem Betätigungsstück 119 ineinander übergehen. Das Federelement 110 ist entsprechend dem Federelement 90 nach Fig. 1 ausgestaltet, das heisst, dass es in der in der Fig. 23 dargestellten Position unter Spannung steht und die beiden Klemmbacken 12 und 51 gegeneinander hält. Durch Druck entsprechend den Pfeilen 98 quer zur Hauptachse der Klemmbacken 12 und 51 können diese sich gegeneinander verschwenken. Es bildet sich - eine Klemmbacke 12 gegenüber der anderen Klemmbacke 51 - eine Winkelbewegung oder Verschwenkbewegung aus, wobei die ursprünglich nicht einander gegenüber liegenden Rillen 13 einander gegenüber verschwenkt werden und sich dabei die beiden Frontflächen der Klemmbacken 12 und 51 voneinander entfernen, um die Stifte 100 einschieben zu können.

Dabei reichen die Enden der Schrauben 16 durch die Klemmbacke 51 in der in der Fig. 24 dargestellten Art und Weise hindurch. In der Klemmbacke 51 sind dementsprechend zwei durchgehende Langlöcher 125 vorgesehen, die von einem konischen oder stufenförmigen Absatz oder Hülsenaufnahme 124 umgeben sind, um die Mutter 21 aufzunehmen. In diesem Fall ist eine Lösung mit einer selbstschneidenden Schraube oder einem Gewinde in dem Loch 125 nicht einsetzbar.

In der Ruhestellung befinden sich die eingesetzten aber nicht angezogenen Schrauben 16 vorzugsweise im Bereich des einen Endes jeden Langloches 125, beispielsweise in der Fig. 24 auf der zeichnerisch rechten Seite. Bei Ausüben des die Klemmbacken 12 und 51 öffnenden Druckes in Pfeilrichtung 98 bewegen sich dann die Schrauben 16 in ihren Hülsen 23 auf die zeichnerisch linke Seite der Fig. 24 und gleichzeitig treten die Hülsen 23 der Muttern 21 tiefer in ihre Aufnahmen 124 beziehungsweise die Schraubenköpfe 18 in ihre Aufnahmen 24 hinein.

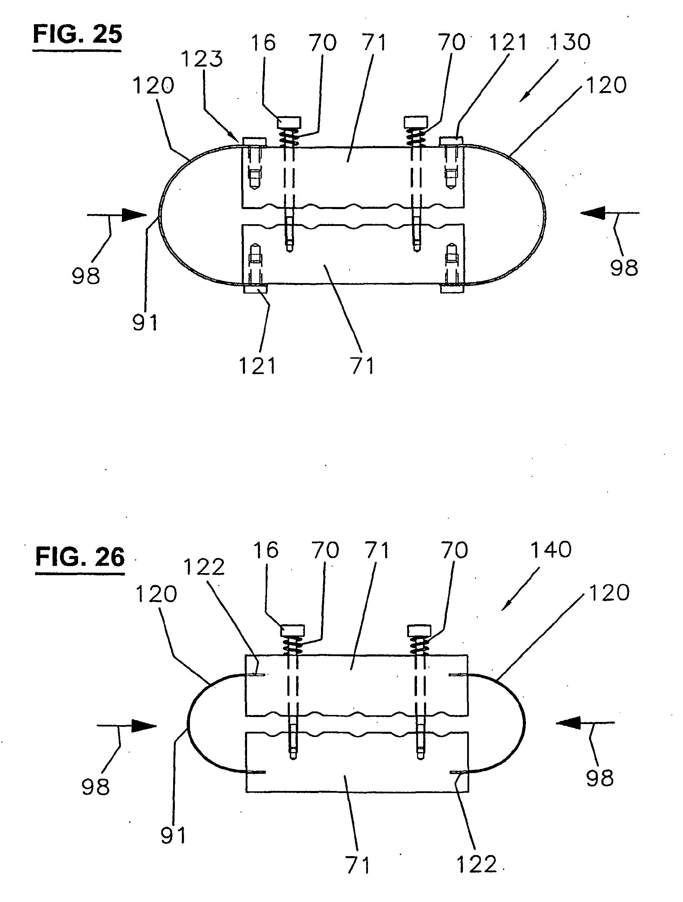

Die Fig. 25 zeigt eine schematische Seitenansicht auf ein sechstes Ausführungsbeispiel eines Klemmelementes 130 gemäss der Erfindung ohne Stifte, und Fig. 26 zeigt eine schematische Seitenansicht auf ein siebtes Ausführungsbeispiel eines Klemmelementes 140 gemäss der Erfindung ohne Stifte. Der Aufbau der Klemmbacken 71 kann jeweils ähnlich zu den Klemmbacken 41 nach Fig. 9 und 10 (mit oder ohne Stab etc.) ausgestaltet sein. Wesentlich ist das Vorsehen von um die Schrauben 16 vorgesehenen Federn 70 zum Zusammendrücken der Klemmbacken 71. In den hier dargestellten Ausführungsbeispielen enden die Schrauben jeweils in einer gewindeten Bohrung oder Hülse der gegenüberliegenden Klemmbacke. Prinzipiell kann die Feder 70 bei einer durchreichenden Schraube 16 auch auf der gegenüberliegenden Seite angeordnet sein und sich zwischen Klemmbacke 71 und Mutter 21 abstützen, was in den Fig. nicht dargestellt ist.

Das Federelement 120 ist in den dargestellten Ausführungsbeispielen der Fig. 25 und 26 eine Blattfeder 120. Diese ist in der Fig. 25 an ihren Enden mit jeweils einem Durchgangsloch versehen, um sie mit Hilfe von Schrauben 121 auf den gegenüberliegenden Enden der Klemmbacken 117 zu fixieren. Durch Druck in Richtung der entgegengesetzt angeordneten Pfeile 98 werden die Blattfedern 120 geöffnet und damit öffnet sich auch der Zwischenraum zwischen den Klemmbacken 71 weiter gegen die Rückstellkraft der Federn 70.

Im Ausführungsbeispiel der Fig. 26 sind die Enden 123 der Blattfedern 120 jeweils in schematisch dargestellte Schlitze 122 eingeschoben, worin sie eingeklebt sein können oder durch die bestehende Vorspannung gehalten werden. Die Enden können flächig sein, oder seitliche oder mittige Spitzen umfassen.

Vorteilhafterweise handelt es sich bei dem Material der Federelemente 90, 80, 60 um weicheres Material als das der Klemmbacken 11, 12, 31, 32, 41, 51, 71. Die Federelemente 90, 80, 60 sind gesteckt und halten durch die inhärente Federkraft in den Aufnahmen 85 oder 95. Sie können in diesen Aufnahmen auch verklemmt oder verklebt oder 2K-gespritzt sein. Gleiches gilt für das Federelement 120, dass eine metallische oder nichtmetallische Blattfeder sein kann.

In den Fig. sind die Federelemente 90, 80, 60, 120 mittig mit dem Federblatt 91 versehen. An den freien Enden des Federblattes 91, die in Lager 85, 95, 122 einzuführen sind, sind entsprechende Lagerzapfen 82, 92 vorgesehen, die sich von einem Steg 83, 93 weg erstrecken. Damit wird das Federelement 90, 80, 60, 120 zwischen Federmontagerippen 28 gehalten. In anderen, hier nicht dargestellten Ausführungsbeispielen, können die besagten Zapfen von aussen in eine einzige mittige oder zwei nebeneinander liegende mittige Federmontagerippen eingeführt werden. Wesentlich ist jeweils, dass das Federblatt 91 einen Bereich umfasst, der vom Benutzer des Klemmelementes erfasst und zusammengedrückt werden kann, um die Klemmbacken auseinander zu bewegen. Es ist festzuhalten, dass die Federelemente 90, 80, 60, 120 keine Kräfte sehen, die von den Stiften 100 auf die Klemmbacken 11, 12, 31, 32, 41, 51 und 71 ausgeübt werden, wenn die Schrauben 16 angezogen sind. Gleiches gilt über allfällige über den Stab 25 eingeleitete Kräfte.

Es ist auch möglich, die beiden Klemmbacken 11, 12, 31, 32, 41, 51, 71 zusammen mit den Federelementen 90, 80, 60, 120 im Spritzguss durch Coinjektion, auch als 2K-Spritzgusstechniken bezeichnet, herzustellen, so dass sich ein einteiliges Klemmelement ergibt. Es ist auch möglich, ein solches einteiliges Klemmelement 90, 80, 60, 120 zusätzlich mit den Federn 70 auszugestalten, wobei dann die Federelemente 90, 80, 60, 120 auch ohne federnde Funktion ausgestaltet sein können.

Aus den Zeichnungen geht hervor, dass einige Klemmbacken 11, 31 und 41 über einen angeformten Stab 25 verfügen, der im Ansatz parallel zu den Verriegelungsschrauben 16 geführt ist. Er kann im weiteren Verlauf noch eine andere Richtung annehmen. Andere Klemmbacken 12, 32, 51, 71 verfügen über keinen solchen Stab 25 und haben an dessen Ansatzort eine Bohrung 115, wie dies im Zusammenhang mit der Fig. 15 beschrieben worden ist. Diese Bohrung 115 kann so ausgestaltet sein, um einen entsprechenden Stab 101 einzusetzen und zu verriegeln.

### Bezugszeichen

- 10: Klemmelement für mehrere Stifte
- 11: Klemmbacke mit Stab
- 12: Klemmbacke
- 13: Rille (Nut)
- 14: Querausnehmungen (Material)
- 15: Bohrung
- 16: Schraube
- 17: Flansch
- 18: Vierkant-Kopf
- 19: Schraubenkörper
- 20: Klemmelement für mehrere Stifte
- 21: Mutter
- 22: Flansch
- 23: Hülse
- 24: Schraubenaufnahme
- 25: angeformter Stab
- 26: Klemmenkörper
- 27: Verstärkungsrippen
- 28: Federmontagerippe
- 29: Anfräsung
- 30: Klemmelement für mehrere Stifte
- 31: Klemmbacke mit Stab
- 32: Klemmbacke
- 33: Bohrung
- 34: Innenbohrung
- 35: Innenkonus
- 36: Innenkonus
- 37: Innenbohrung
- 40: Klemmelement für mehrere Stifte
- 41: Klemmbacke mit Stab
- 50: Klemmelement für mehrere Stifte
- 51: Klemmbacke mit Stab und Langloch
- 60: Federelement
- 61: Verstärkungsrippe
- 70: Spiralfeder
- 71: Klemmbacke
- 73: Pfeilrichtung
- 80: Federelement
- 82: Rechteckzapfen
- 83: Pfeilrichtung
- 85: Schlitz
- 89: Betätigungsbereich
- 90: Federelement
- 91: Federblatt
- 92: Zapfen
- 93: Querrippe
- 94: Steg
- 95: Federzapfenloch
- 96: Längsrippe
- 97: Lochführung
- 98: Pfeilrichtung
- 99: Betätigungsbereich
- 100: Stift
- 101: ansetzbarer Stab
- 102: Befestigungsschraube für den ansetzbaren Stab
- 103: zylindrisches Schraubgewinde
- 104: konischer Schraubenkopf
- 105: Innengewinde
- 106: Absatz
- 107: Flansch
- 108: Ansatz
- 109: Sicherungsschraube
- 110: Federelement
- 111: erster schmalerer Abschnitt
- 112: zweiter breiterer Abschnitt
- 113: Schulter
- 114: Gewinde
- 115: Innenbohrung
- 116: konische Mündung
- 117: längeres Bein
- 118: kürzeres Bein
- 119: Betätigungsstück
- 120: Feder
- 121: Schraube
- 122: Schlitz
- 123: freie Enden
- 124: Hülsenaufnahme
- 125: Langloch
- 130: Klemmelement für mehrere Stifte
- 140: Klemmelement für mehrere Stifte

## Patentansprüche

1. Klemmelement (10, 20, 30, 40, 50) zur Stabilisierung von Knochenbrüchen zum Klemmen von mindestens zwei stabförmigen Elementen (100), mit zwei einander gegenüberliegenden Klemmbacken (11, 12, 31, 32, 41, 51, 71), die jeweils über mindestens zwei Rillen (13) verfügen, zu denen jeweils entsprechende Rillen (13) in der gegenüberliegenden Klemmbacke (11, 12, 31, 32) korrespondieren, jedes Rillenpaar jeweils geeignet zur Aufnahme eines der stabförmigen Elemente (100), und mit mindestens zwei Federelementen (60, 70, 80, 90, 110, 120), die auf die gegenüberliegenden Klemmbacken (11, 12, 31, 32) wirken, wobei in den Klemmbacken (11, 12, 31, 32, 41, 51, 71) jeweils mindestens eine durchgehende Bohrung (15, 125) vorgesehen ist, die senkrecht zur Ebene der aneinandergrenzenden Klemmbacken (11, 12, 31, 32, 41, 51, 71) und fluchtend zueinander ausgerichtet sind und die geeignet sind, ein Verriegelungselement (16, 21) aufzunehmen, **dadurch gekennzeichnet, dass** die Federelemente (60, 70, 80, 90, 110, 120) ausgestaltet sind, um sowohl ohne eingelegtes stabförmiges Element (100) als auch bei einem oder mehreren eingelegten stabförmigen Elementen (100) die besagten Klemmbacken (11, 12, 31, 32, 41, 51, 71) in Richtung ihrer Schliessung vorzuspannen.

2. Klemmelement nach Anspruch 1, **dadurch gekennzeichnet, dass** das Verriegelungselement (16, 21) aus einer in die Bohrung der einen Klemmbacke (11, 12, 31, 32, 41, 51, 71) einsetzbaren Schraube (16) und einer komplementären innen gewindeten Mutter (21) besteht, die in die andere Klemmbacke (11, 12, 31, 32, 41, 51, 71) einsetzbar ist, so dass das Klemmelement durch Eindrehen der Schraube in die Mutter (21) blockierbar ist.

3. Klemmelement nach Anspruch 1, **dadurch gekennzeichnet, dass** das Verriegelungselement (16, 21) eine in die Bohrung der einen Klemmbacke (11, 12, 31, 32, 41, 51, 71) einsetzbare Schraube (16) umfasst, die in die andere Klemmbacke (11, 12, 31, 32, 41, 51, 71) einschraubbar ist, wobei die andere Klemmbacke (11, 12, 31, 32, 41, 51, 71) über ein entsprechendes Innengewinde verfügt oder die einsetzbare Schraube (16) eine selbstschneidende Schraube ist.

4. Klemmelement nach einem der Ansprüche 1 bis 3, **dadurch gekennzeichnet, dass** die Klemmbacken (11, 12, 31, 32) an ihren seitlichen äusseren Enden, an denen keine stabförmige Elemente (100) herausragen, über Lager (85, 95, 122) verfügen, in denen Enden (123) oder Verbindungszapfen (92, 82) der Federelemente (60, 80, 90, 110, 120) gesteckt oder verklemmt oder 2K-gespritzt werden können.

5. Klemmelement nach Anspruch 4, **dadurch gekennzeichnet, dass** die Klemmbacken (11, 12, 31, 32) über Federmontagerippen (28) verfügen, in denen Lagerausnehmungen (85, 95) für Verbindungszapfen (92, 82) vorgesehen sind.

6. Klemmelement nach Anspruch 4 oder 5, **dadurch gekennzeichnet, dass** die Federelemente (60, 80, 90, 110, 120) über ein C-förmiges Federblatt (91) verfügen, das an seinen freien Enden vorteilhafterweise die Verbindungszapfen (92, 82) aufweist, und dass das Federblatt (91) mittig einen direkten flächigen Betätigungsbereich (89, 99, 119) oder einen über Rippen (61) realisierten Betätigungsbereich aufweist.

7. Klemmelement nach einem der Ansprüche 4 bis 6, **dadurch gekennzeichnet, dass** die Federelemente (60, 80, 90, 110, 120) und die Klemmbacken (11, 12, 31, 32, 41, 51, 71) aus Kunststoff bestehen, wobei der Kunststoff der Federelemente (60, 80, 90, 110, 120) weicher oder flexibler als der Kunststoff der Klemmbacken (11, 12, 31, 32, 41, 51, 71) ist.

8. Klemmelement nach einem der Ansprüche 4 bis 6, **dadurch gekennzeichnet, dass** mindestens ein Federelement (110) asymmetrisch ausgestaltet ist und ein längeres (117) und ein kürzeres (118) Bein aufweist, die über einen Betätigungsbereich (119) miteinander verbunden sind.

9. Klemmelement nach Anspruch 8, **dadurch gekennzeichnet, dass** die durchgehenden Bohrungen in einem Klemmelement (51) jeweils durchgehende Langlöcher (125) zur Aufnahme der Muttern (21) sind.

10. Klemmelement nach einem der Ansprüche 1 bis 3, **dadurch gekennzeichnet, dass** mindestens zwei durchgehende Bohrungen (15) für zwei Verriegelungselemente (16, 21) vorgesehen sind, dass die Federelemente (70) Spiralfedern oder mit einer mittigen Bohrung versehene Federn sind, die um die Schäfte (19) der Verriegelungselemente (16, 21) angeordnet sind und sich jeweils gegen die Klemmbacke (24 an 41) und einen Flanschabschnitt (18) des Schaftes (19) des Verriegelungselementes (16, 21) abstützen.

11. Klemmelement nach Anspruch 10, **dadurch gekennzeichnet, dass** ein zusätzliches Federelement (120) vorgesehen ist, das an seinen freien Enden (123) oder Verbindungszapfen (92, 82) mit den Klemmbacken (71) verbunden ist, und dass das zusätzliche Federelement (120) mittig einen direkten flächigen Betätigungsbereich oder einen über Rippen (61) realisierten Betätigungsbereich aufweist.

12. Klemmelement nach einem der Ansprüche 1 bis 11, **dadurch gekennzeichnet, dass** die Rillen (13) parallel zueinander ausgerichtet sind oder dass verschiedene Rillen paarweise in einem Winkel zueinander stehen.

13. Klemmelement nach einem der Ansprüche 1 bis 12, **dadurch gekennzeichnet, dass** die Klemmbacken (11, 12, 31, 32, 41, 51, 71) über im aufeinander zu gerichteten Mündungsbereich (37) der Bohrung (15) über drei oder mehr Zentrierrippen für das Verriegelungselement verfügen.

## Claims

1. A clamping element (10, 20, 30, 40, 50) for stabilising bone fractures for the clamping of at least two rod-shaped elements (100) comprising a pair of opposing jaws (11, 12, 31, 32, 41, 51, 71) that have at least two grooves (13) corresponding to respective grooves (13) in the opposing jaws (11, 12, 31, 32), each pair of grooves being adapted for the acceptance of one of the rod-shaped element (100), and spring elements (60, 70, 80, 90, 110, 120) acting upon the opposing jaws (11, 12, 31, 32), wherein at least one through bore (15, 125) is provided in each of the jaws (11, 12, 31, 32, 41, 51, 71), which bores are vertical to the level of the adjoining jaws (11, 12, 31, 32, 41, 51, 71) and are aligned to one another and suitable to the acceptance of a locking element (16, 21), **characterized in that** the spring elements (60, 70, 80, 90, 110, 120) are adapted to tense the jaws (11, 12, 31, 32, 41, 51, 71) in their closing direction in absence of an inserted rod-shaped element (100) as well as in the case of one or more inserted rod-shaped elements (100).

2. The clamping element according to claim 1, **characterized in that** the locking element (16, 21) consists of a screw that may be inserted into the bore of one jaw (11, 12, 31, 32, 41, 51, 71) and a complementary interior-threaded nut (21) that can be inserted into the other jaw (11, 12, 31, 32, 41, 51, 71), so that the clamping element can be locked by turning the crew in the nut (21).

3. The clamping element according to claim 1, **characterized in that** the locking element (16, 21) includes a screw (16) which can be inserted into the bore of a jaw (11, 12, 31, 32, 41, 51, 71), and which can be screwed into the other jaw (11, 12, 31, 32, 41, 51, 71), whereby the other jaw (11, 12, 31, 32, 41, 51, 71) has a corresponding interior threading or the insertable screw (16) is a self-tapping screw.

4. The clamping element according to any one of claims 1 to 3, **characterized in that** the jaws (11, 12, 31, 32) have mounts (85, 95, 122) on their lateral ends, from which no rod-shaped elements (100) protrude, into which ends (123) or connecting plugs (92, 82) of the spring elements (60, 80, 90, 110, 120) may be stuck or glued or 2K-injected.

5. The clamping element according to claim 4, **characterized in that** the jaws (11, 12, 31, 32) have spring-mounted, ribs (28), in which the recessed mounts (85, 95) are provided for connection plugs (92, 82).

6. The clamping element according to claim 4 or 5, **characterized in that** the spring elements (60, 80, 90, 110, 120) have a c-shaped leaf spring (91) that preferably exhibits connecting knobs (92, 82) on its free ends, and that the leaf spring (91) exhibits at its center a direct flat manipulation area (89, 99, 119) or an activation area that is realized through ribs (61).

7. The clamping element according to any one of claims 4 to 6, **characterized in that** the spring elements (60, 80, 90, 110, 120) and the jaws (11, 12, 31, 32, 41, 51, 71) consist of plastic, whereby the plastic of the spring elements (60, 80, 90, 110, 120) is softer or more flexible than the plastic of the jaws (11, 12, 31, 32, 41, 51, 71).

8. The clamping element according to any one of claims 4 to 6, **characterized in that** the spring element (110) is asymmetrically shaped and exhibits a longer leg (117) and a shorter leg (118), that are connected to each other through a manipulation area (119).

9. The clamping element according to claim 8, **characterized in that** the through bores in a clamping element (51) are each through oblong holes (125) for the acceptance of the nuts (21).

10. The clamping element according to any one of claims 1 to 3, **characterized in that** at least two through bores (15) are provided for two locking elements (16, 21) and **in that** the spring elements (70) are spiral springs or are springs provided with a center bore, that are arranged around the shanks (19) of the locking elements (16, 21) and are each supported against the jaws (24=>41) and a flange section (18) of the shank (19) of the locking element (16, 21).

11. The clamping element according to claim 10, **characterized in that** an additional spring element (120) is provided, that, by its free ends (123) or connecting plugs (92, 82) is connected to the jaws (71), and that the additional spring element (120) exhibits at its center a direct flat manipulation area or an activation area that is realized through ribs (61).

12. The clamping element according to any one of claims 1 to 11, **characterized in that** the grooves (13) are set parallel to each other or that various grooves are in pairs at an angle to one another.

13. The clamping element according to any one of claims 1 to 12, **characterized in that** the jaws (11, 12, 31, 32, 41, 51, 71) have three or more centering ribs that can be directed on top of each other in an aperture area (37) of the bore (15) for the locking element.

## Revendications

1. Elément de serrage (10, 20, 30, 40, 50) pour stabiliser
des fractures d'os pour accrocher un sangle au moins deux tiges ou éléments en forme de bâton (100), avec deux mâchoires (11, 12, 31, 32, 41, 51, 71) opposantes qui possèdent au moins deux rainures (13) correspondantes à des rainures (13) dans la mâchoire (11, 12, 31, 32) opposante, où chaque paire de rainures est adaptée pour accepter un des éléments de tige (100), et avec au moins deux éléments élastiques (60, 70, 80, 90, 110, 120) qui agissent sur les mâchoires (11, 12, 31, 32) opposantes, où au moins un alésage (15, 125) continu est prévu dans chacune des mâchoires (11, 12, 31, 32, 41, 51, 71), qui sont alignés verticalement au plan des mâchoires (11, 12, 31, 32, 41, 51, 71) avoisinantes et en alignement un par rapport à l'autre, et qui sont adaptés d'accepter un élément de verrouillage (16, 21), **caractérisé en ce que** les éléments élastiques (60, 70, 80, 90, 110, 120) sont adaptés pour s'accoupler dans la direction de leur fermeture aussi bien sans un élément de tige (100) installé ou lors de l'installation d'un ou de plusieurs éléments de tige (100).

2. Elément de serrage selon la revendication 1, **caractérisé en ce que** l'élément de verrouillage (16, 21) dispose d'une vis (16) qui peut être introduite dans l'alésage d'une mâchoire (11, 12, 31, 32, 41, 51, 71), et d'un écrou (21) complémentaire et taraudé à l'intérieur, qui peut être introduit dans l'autre mâchoire (11, 12, 31, 32, 41, 51, 71) pour bloquer l'élément de serrage par introduction de la vis dans l'écrou (21).

3. Elément de serrage selon la revendication 1, **caractérisé en ce que** l'élément de verrouillage (16, 21) comprend une vis (16) qui peut être introduite dans l'alésage d'une mâchoire (11, 12, 31, 32, 41, 51, 71) qui peut être vissée dans l'autre mâchoire (11, 12, 31, 32, 41, 51, 71), où l'autre mâchoire (11, 12, 31, 32, 41, 51, 71) possède un tel taraudage intérieur ou la vis (16) et une vis autotaraudeuse.

4. Elément de serrage selon une des revendications 1 à 3, **caractérisé en ce que** les mâchoires (11, 12, 31, 32) possèdent à leur bouts extérieurs latéraux des crochets (85, 95, 122) dans lesquels des bouts (123) ou des faussets de connexion (92, 82) des éléments élastiques (60, 80, 90, 110, 120) peuvent être mis ou coincés ou collés par injection de deux composantes plastique.

5. Elément de serrage selon la revendication 4, **caractérisé en ce que** la mâchoire (11, 12, 31, 32) possède des nervures de montage des ressort (28), dans lesquels les crochets (85, 95) pour les faussets de connexion (92, 82) sont prévus.

6. Elément de serrage selon la revendication 4 ou 5, **caractérisé en ce que** les éléments élastiques (60, 80, 90, 110, 120) possèdent une lamelle de ressort en forme de C, qui possède à ses bouts libres préférablement les faussets de connexion (92, 82) et **en ce que** la lamelle de ressort (91) comprend au centre une zone de manipulation (89, 99, 119) directe et plate ou une zone de manipulation comprenant des côtes (61).

7. Elément de serrage selon une quelconque des revendications 4 à 6, **caractérisé en ce que** les éléments élastiques (60, 80, 90, 110, 120) et les mâchoires (11, 12, 31, 32, 41, 51, 71) sont de matière plastique, ou la matière plastique des éléments élastiques (60, 80, 90, 110, 120) est plus mou ou plus flexible que la matière plastique des mâchoires (11, 12, 31, 32, 41, 51, 71).

8. Elément de serrage selon une quelconque des revendications 4 et 6, **caractérisé en ce qu'**au moins un élément élastique (110) est formé d'une manière asymétrique et possède une jambe plus longue (117) et une jambe plus courte (118), qui sont reliées par la zone de manipulation (119).

9. Elément de serrage selon la revendication 8, **caractérisé en ce que** les alésages continus dans un élément de serrage (51) sont des trous oblongs (125) pour accepter les écrous (21).

10. Elément de serrage selon une quelconque des revendications 1 à 3, **caractérisé en ce qu'**au moins deux alésages (15) continus sont prévus pour les deux éléments de verrouillage (16, 21), **en ce que** les éléments élastiques (70) sont des ressorts en spirale ou des ressorts avec un trou central, qui sont arrangés autour des manches (19) des éléments de verrouillage (16, 21) et s'appuient contre la mâchoire (24=>41) et une section de bride (18) de la manche (19) de l'élément de verrouillage (16, 21).

11. Elément de serrage selon la revendication 10, **caractérisé en ce qu'**un élément élastique supplémentaire (120) est prévu, qui est relié à ses bouts libres (123) ou faussets de connexion (92, 82) avec les mâchoires (71), et **en ce que** l'élément élastique (120) supplémentaire possède au centre d'une zone de manipulation directe et plate ou une zone de manipulation réalisée par des côtes (61).

12. Elément de serrage selon une quelconque des revendications 1 à 11, **caractérisé en ce que** les rainures (13) sont disposées les uns parallèles aux autres ou que des différentes rainures sont arrangées dans un angle par paires.

13. Elément de serrage selon une quelconque des revendications 1 à 12, **caractérisé en ce que** les mâchoires (11, 12, 31, 32, 41, 51, 71) possèdent dans l'embouchure (37) de l'alésage (15) trois côtes ou plus pour centrer l'élément de verrouillage.
